# EUROPEAN PATENT SPECIFICATION

(11) **EP 1 209 225 B1**
(45) Date of publication and mention of the grant of the patent: **18.11.2009**
(21) Application number: 01402969.8
(22) Date of filing: 20.11.2001
(51) Int. Cl.: C12N 1/18, A21D 8/04

(54) **New baker's yeasts and strains for their preparation**
Neue Stämme der Bäckerhefe
Nouvelles souches de levure de panification

(30) Priority: 20.11.2000 US 716639
(43) Date of publication of application: 29.05.2002
(73) Proprietor: LESAFFRE et Cie, F-75001 Paris (FR)
(72) Inventor: Colavizza, Didier, 59163 Conde sur l'Escaut (FR); Deniaud, Arnaud, 59700 Marcq en Baroeul (FR)
(74) Representative: Touati, Catherine

(56) References cited:
- US-A- 5 801 049
- NAKAGAWA AND OUCHI: "Construction from a Single Parent of Baker's Yeast Strains with High Freeze Tolerance and Fermentative Activity in Both Lean and Sweet Doughs" APPLIED AND ENVIRONMENTAL MICROBIOLOGY, WASHINGTON,DC, US, vol. 60, no. 10, October 1994 (1994-10), pages 3499-3502, XP002119705 ISSN: 0099-2240
- MEADEN P: "PADding out the POF gene" FERMENT, vol. 7, no. 4, 1994, pages 229-230, XP002189235
- VAN ROIJEN R., KLAASSEN P.: "Genetic modification in the food industry: a strategy for food quality improvement" 1998 , BLACKIE , LONDON XP001056177 * page 158 - page 173 *

## Description

The invention relates to new baker's yeasts or bread-making yeasts and baker's yeast strains, to methods and processes for their preparation and to the use of the new baker's yeasts in the preparation of doughs and baked products.

In the USA, the trade of frozen doughs, notably of frozen sweet doughs, such as frozen doughs intended for bakery products called "Rolls" or for Danish sweet pastries or for similar sweet fermented and baked products is rapidly expanding. However, the freezing of the yeast-containing dough involves an important stress for the yeast.

In the USA, small breads or fermented pastries are often aromatized or flavored with the spice cinnamon. This spice contains the chemical compounds cinnamic acid and cinnamaldehyde. These chemical compounds can be metabolized by yeasts, which leads to the appearance of bad taste and of bad flavors, also called "off-flavors".

Baker's yeasts which are resistant to the stress caused by freezing or deep-freezing are already known. Such yeasts can be used in the preparation of frozen doughs without the necessity to increase the amount of yeast to an important extent, as compared to the amount of yeast required in the preparation of doughs of the same type which are not subjected to freezing before being baked. Such an increase in the amount of yeast used is necessary when preparing frozen doughs using conventional baker's yeasts which are not resistant to said stress. Baker's yeasts resistant to freezing have been developed in Europe and Japan.

Also known are baker's yeasts which can be used without formation of off-flavors in the manufacture of cinnamon-containing bakery products. Such baker's yeasts, which do not give rise to off-flavors in the presence of cinnamon, are commonly marketed in the USA.

According to the present invention, new baker's yeasts have been developed which combine the properties of the two types of baker's yeasts here-above discussed.

These new baker's yeasts :
- have good general performances in not-delayed bread-making processes,
- are resistant to the stress caused by freezing when they are used in sweet doughs and,
- do not give rise to the appearance of bad taste or of off-flavors in the presence of cinnamon.

Indeed, the property of not giving rise to the appearance of bad taste or of off-flavors is essential for a baker's yeast and must be verified in any bread-making process wherein the said baker's yeast is intended to be used.

In the context of the present invention:
- the term "bread-making processes" refers to processes for the preparation of baked products, such as bread, rolls, buns and pastries, by means of baking a yeast-fermented dough;
- the term "not-delayed bread-making processes" refers to bread-making processes whereby the yeast-containing dough is not subjected to freezing or deep-freezing before being baked;
- the term "sweet dough" refers to a dough containing added natural sweeteners, such as, for example, sugar and/or high fructose corn syrup.

Yeasts having good general performances in not-delayed bread-making processes are baker's yeasts presenting the properties which, according to the baker, make that the said baker's yeasts are suitable for use as a leavening agent in not-delayed bread-making processes. Such baker's yeasts therefore correspond to the baker's yeasts having the properties which condition the baker's decision to buy the said baker's yeast.

The good general performances of the above yeasts according to the present invention in not-delayed bread-making processes can be illustrated by means of the tests A₁, A₅ and A₆ which are carried out with the fermentometer of Burrows and Harrison, and which are described in column 5 of US patent No. 5,741,695 of April 21, 1998.

### Test A₁ (fresh compressed yeast)

An amount of compressed yeast corresponding to a yeast solids content, i.e. a yeast dry matter content of 160 mg is added to 20 g of flour incubated at 30°C, this yeast having been mixed with 15 ml of water containing 27 g of NaCI per liter and 4 g of SO₄(NH₄)₂ per liter. The components are mixed with the aid of a spatula for 40 seconds to form a paste which is placed on a waterbath adjusted to 30°C. 13 Minutes after the onset of mixing, the vessel containing the paste is hermetically sealed. The total quantity of gas produced is measured after 60 minutes and then after 120 minutes. This quantity is expressed in ml at 30°C and under 760 mm of Hg.

For all the yeasts likely to show in 120 minutes a gas development equal to or more than 150 ml, the amount of fermentable sugars solely present in the flour is insufficient and is a limiting factor, so that the test is modified as indicated hereinafter: an amount of compressed yeast corresponding to a yeast solids content of 106 mg is used instead of an amount corresponding to a yeast solids content of 160 mg, and the reading of the quantity of gas produced is by convention multiplied by 1.5.

### Test A₅ (fresh compressed yeast)

Test identical to test A₁, but with the addition of 4 g of sucrose to the flour. The total quantity of gas produced is measured after 60 minutes and after 120 minutes.

### Test A₆ (fresh compressed yeast)

The same procedure as in test A₁, but with an amount of compressed yeast corresponding to a yeast solid content of 320 mg and with 25g of flour (instead of 20g of flour) incubated at 30°C to which 6.5g of icing sugar has been added. The total quantity of gas produced is measured after 60 minutes and after 120 minutes.

In the said tests A₁, A₅ and A₆, the said good general performances of the said new baker's yeasts in not-delayed bread-making processes lead to the following total quantities of gas produced:
- in test A₁, at least 150 ml in 2 hours,
- in test A₅, at least 90 ml in 2 hours,
- in test A₆, at least 80 ml in 2 hours.

In other words, the said new baker's yeasts are characterized by the fact that, in not-delayed bread-making processes, they give rise in the fermentometer tests A₁, A₅ and A₆ carried out with the fermentometer of Burrows and Harrison to quantities of gas produced which are at least equivalent (i.e. substantially equal or equal) to the quantities of gas produced under the same circumstances by a control yeast produced according to a conventional process starting from the strain deposited at the "Collection Nationale de Cultures de Microorganismes" (CNCM), Institut Pasteur under the number CNCM I-2412, said strain being representative of the strains commonly used in the USA for the manufacture of baker's yeasts.

A conventional process of baker's yeast manufacture is a process described in the chapter 6 "Baker's Yeast Production" of the handbook "Yeast Technology", Second Edition, Reed and Nagodawithana, An Avi Book published by Van Nostrand Reinhold, 1991.

The resistance of the said baker's yeasts according to the invention to the stress caused by the freezing of the yeast-containing dough is illustrated :
- by the fact that, when the said new yeasts are used in the preparation of doughs corresponding to formulations of sweet Danish pastries, whereby the yeast-containing doughs are subjected to freezing during at least 100 days and then thawed, the total amount of gas released by the said new yeasts are at least 20 %, preferably at least 30 % and still more preferably at least 40 % higher than the total amount of gas released under the same circumstances by a control yeast obtained starting from a control strain such as the strain CNCM I-2412 by means of a conventional process of baker's yeast manufacture,
- by the fact that, when the said new yeasts are used in the above-defined use, the proof time of the said doughs corresponding to formulations of sweet Danish pastries, after having been subjected to freezing during at least 100 days and then thawed, is at least 10 %, preferably at least 15 % and still more preferably at least 20 % shorter than the proof time measured under the same circumstances when using the above-defined conventional control yeast.

Preferably, the said total amount of gas released by the yeasts in the doughs is measured during 2 hours and 30 minutes at 27°C using the zymotachygraphe CHOPIN^{®} and the proof times are preferably measured at 35°C.

Doughs corresponding to dough formulations for sweet Danish pastries are doughs comprising about 18% by weight of sugar (sucrose) with respect to the weight of flour used and further comprising fats. Such sweet-Danish-pastry doughs typically comprise from 10 to 20% by weight of fats with respect to the weight of flour used.

For the purpose of the above comparative tests, the new yeast and the control yeast must naturally be used in the same form and in the same amounts.

It is pointed out that, according to the handbook of basic technical baking terminology by E.J. Pyler, the proof time is the length of time for which a moulded dough piece is held in the final proofer prior to baking so it can attain the desired degree of aeration or volume increase.

The zymotachygraphe CHOPIN^{®} or CHOPIN^{®} zymotachygraphe is a conventional apparatus known to those skilled in the art for measuring the total gaseous release of a dough ball or piece. This apparatus is described in a detailed manner in chapter VII B "Appreciation du pouvoir fermentaire" (appreciation of the fermenting power), §6.5 "Le Zymotachygraphe" (CHOPIN, 1973), pages 461 to 463 of the manual "Guide pratique d'analyses dans les industries des céréales", B. Godon and W. Loisel, Technique et Documentation (Lavoisier) 1984, ISBN 2-85206-081-7 Collection 2-85206-230-5.

The fermentometer of Burrows and Harrison is the object of §6.1 of Chapter VIIB, pages 454 to 460, of said manual.

For the preparation of the said new baker's yeasts according to the invention, it is possible to use two yeast strains which were deposited on the 24^{th} March 2000 according to the Budapest Convention with the "Collection Nationale de Cultures de Microorganismes" (CNCM), Institut Pasteur, 28 rue du Docteur Roux, 75724 PARIS CEDEX 15, FRANCE, under the numbers:
I-2421 (strain L17)
I-2422 (strain L35).
Strain L17 deposited under the number CNCM I-2421 has, in a different context, been mentioned but not disclosed as such in US patent No. 5,741,695. Strain L35, deposited under the number CNCM I-2422 was obtained by mutation starting from strain L17.

These two yeast strains were selected from a large number of strains, including the strains of the private collection of the Lesaffre Group, using three systematic selection tests. The confidential and private collection of the Lesaffre Group contains numerous strains which were constructed, notably according to the reproducible construction processes of new strains disclosed by the US patents Nos. 4,396,632 and 5,741,695.

In the said selection tests, the yeast strain deposited with the "Collection Nationale de Cultures de Microorganismes", under the number I-2412 was used as control yeast strain. Said control yeast strain is representative of the strains which are commonly used in the USA for the manufacture of baker's yeasts, and in particular for the manufacture of baker's yeasts intended for the preparation of products such as sweet rolls and Danish pastries.

The control strain and the various tested strains were cultivated on cane molasses in pilot installations. The scheme of the cultivation on cane molasses used was the one disclosed in example 3 of US patent 5,741,695, from column 12, line 40, to column 13, line 26. Fresh baker's yeasts having a dry matter content of about 32% were obtained. The nitrogen content with respect to the dry matter of these fresh yeasts was adjusted to between about 8.2% and about 8.5%.

The first selection test consists in selecting those fresh baker's yeasts that give rise to the following total quantities of gas released in tests A₁, A₅ and A₆ carried out with a fermentometer of Burrows and Harrison as defined above:
- in test A₁: at least 150 ml in two hours,
- in test A₅: at least 90 ml in two hours, and
- in test A₆: at least 80 ml in two hours.

It may be pointed out that all these results, in absolute values, concerning gas releases or proof times, must always be compared with respect to at least one control. As a matter of fact, the principles indicated in example 6 of US patent 5,741,695, columns 19 and 20, and especially column 20, lines 50 to 57, generally apply.

The second selection test consists in examining the behavior with respect to cinnamon of the fresh yeasts issued from the first selection test, i.e. those fresh yeasts, which, in the tests A₁, A₅ and A₆, give the results defined hereabove.

In the second selection test, an amount of the said selected fresh baker's yeasts corresponding to 150 mg of yeast dry matter is used to ferment 20 ml of each of two sweet nutrient solutions during 4 hours at 30°C, under weak agitation in 125 ml flasks not hermetically closed. The basic nutrient solution, buffered at pH 5.5, contains in a total volume of 1000 ml: 4.7 g (NH₄)₂HP0₄, 2 g Mg SO₄7H₂O, 0.8 g KCL, 10 ml vitamin solution and 150 ml citrate buffer. The 10 ml vitamin solution contains 4 mg thiamin (B1 vitamin), 4 mg pyridoxyn (B6 vitamin) and 40 mg nicotinic acid. The 150 ml citrate buffer contains 14.14 g of trisodium citrate and citric acid necessary to adjust the pH to 5.5. The first sweet nutrient solution or control solution consists of the said basic nutrient solution to which 6% of sorbitol, 0.25% of yeast extract and 6% of glucose have been added. The second sweet nutrient solution consists of the said basic nutrient solution to which 6% of sorbitol, 0.25% of yeast extract, 6% of glucose and 0.04% of cinnamic acid have been added.

The above percentages are expressed in weight with respect to the total or final volume of sweet nutrient solutions, i.e. 1% corresponds to 1g / 100ml.

The second selection test consists in comparing the odor of the solution fermented without cinnamic acid. (first sweet nutrient solution or control solution) with that of the solution fermented in the presence of cinnamic acid (second sweet nutrient solution). This test of comparison of odors, based on the detection of off flavors due to the decomposition of cinnamic acid is made on the basis of notes given by a jury. The notes given by the jury can be confirmed by analyses of the decomposition rate of cinnamic acid, i.e. by the determination of the cinnamic acid still present at the end of the test and/or by the determination of styrene present in the solution fermented in the presence of cinnamic acid.

Cinnamic acid and styrene can be detected and their concentration can be measured using chromatographic methods known to those skilled in the art.

The following method may in particular be used for determining the amount of cinnamic acid present in the solutions. The fermented solutions are first centrifuged at 4°C in order to remove yeast cells. The amount of cinnamic acid present in the resulting solution is dosed by reverse phase HPLC (High Performance Liquid Chromatography) on C18 column. The elution of the column is realized by a gradient of acetonitrile between 10% and 40% in water, in presence of 0.1% of trifluoroacetic acid (percentages volume/volume). The detection of cinnamic acid is realized by an U.V. detector at 260 nm.

The amount of styrene present in the solutions can be determined by gas chromatography coupled with mass spectrometry. The amount of styrene present in the solutions is, for example, determined by means of a VARIAN® GC 38000 gas chromatograph equipped with a column CHROMPACK® CP-Wax 52 CB 30m*0.25mm, df:0.5µm. Sampling can be done using a static headspace, 3 g of supernatant being placed into 10 ml glass vials which are heated at 35°C with agitation for the timed equilibrium step (15 min), the headspace volume of 100 µl being injected into the GC column. The oven temperature of the chromatograph is programmed as follows: 2 min isotherm at 55°C, heating at 5°C/min up to 230°C. Helium is used as carrier gas with a flow rate of 1 ml/min. The styrene is detected by mass spectrometry using a SATURN 2000 VARIAN® mass spectrometer.

This second test shows that most of the baker's yeasts used in Europe, and especially the baker's yeasts which in Europe are considered as efficient on frozen doughs, give rise to the appearance of off-flavors in the presence of cinnamic acid.

At the end of the second selection test, only baker's yeasts which do not give rise to the formation of off-flavors in the presence of cinnamic acid are selected. Baker's yeasts obtained from the strain L17 (CNCM I-2421) and from the strain L35 (CNCM I-2422) pass this second selection test successfully.

The control baker's yeast obtained starting from the strain CNCM I-2412 also does not give rise to the formation of off-flavors in the second selection test.

The strains CNCM I-2412, CNCM I-2421 and CNCM I-2422 are three examples of strains which pass this second selection test successfully. They may consequently be used as comparative or reference strains in the calibration of this biological test.

The strains selected by means of the said first and second selection tests are subjected to a third selection test. The said third selection test consists in determining the resistance of the selected strains against the stress caused by freezing, in other words their qualification or suitability for the preparation of baker's yeasts which can be used in the manufacture of frozen yeast-containing doughs , and in particular of such frozen doughs for sweet rolls and for sweet Danish pastries.

The compositions of the sweet-roll dough and the sweet-Danish-pastry dough used in the third selection test are as follows:
1) Sweet-roll dough containing 6 % of HFCS (high fructose corn syrup) dry matter or 10 % of sucrose:
   - flour 100.0
   - water 55.0 %
   - yeast expressed in terms of yeast dry matter 1.86 %
   - HFCS expressed in terms of its dry matter 6.0 %
      (or sucrose 10.0 %)
   - fat 5.0 %
   - salt 2.0 %
   - dough improver 2.0 %
2) Sweet Danish pastries:
   - flour 100.0
   - water 46.0 %
   - yeast expressed in terms of yeast dry matter 2.72 %
   - sucrose 18.0 %
   - fat 13.0 %
   - pulverulent lactoserum 4.0 %
   - salt 2.0 %
   - dough improver 2.0 %

The percentages are expressed in what is called "baker's percent", that is to say in parts by weight per 100 parts by weight of flour.

The flour used is a US flour having a high gluten content well adapted to bread-making processes comprising a deep-freezing step.

The dough improver used for the rolls and for the Danish pastries provides gluten, diacetyltartaric esters of monoglycerides (DATEM), ascorbic acid, alpha-amylases and hemicellulases, in amounts permitting to obtain optimized dough pieces for deep frozen and long storages at -20°C.

The conditions of the manufacture of the frozen doughs intended for rolls and for Danish pastries, those of the storage of the frozen doughs until thawing of the frozen doughs and those of the evaluation tests to which the dough pieces are subjected are as follows:
- mixing (= kneading),
- temperature of the dough at the end of mixing: 19°C,
- separation in balls or dough pieces of 100 g, in a room the temperature of which is 19°C,
- beginning of deep-freezing 35 minutes after the end of mixing,
- deep-freezing during 35 minutes at -30°C which provides a temperature at the center of dough pieces of -5°C,
- storage at -20°C during 100 days,
- thawing within 20 hours at 0°C at the end of each of the storage period of 100 days,
- determination after thawing of the total amount of gas released by the yeast in the dough pieces during 2 hours and 30 minutes at 27°C using the zymotachygraphe Chopin^{®},
- proofing and measuring of the proof time at 35°C on three dough pieces or balls per yeast strain,
- baking and appreciation of volume and of the scoring of obtained rolls or pastries and verification of the absence of any bad taste or any off-flavors.

Within the framework of the third selection test, baker's yeasts are selected which provide dough pieces of sweet Danish pastries obtained as here-above disclosed and thawed after having been stored for 100 days at - 20°C, which showed the following performances:
- total amount of gas released measured using a zymotachygraphe CHOPIN^{®} in 2 hours and 30 minutes at 27°C at least 20 % higher than the total amount of gas released measured under the same test conditions using the control yeast manufactured starting from the strain CNCM I-2412 by means of a conventional process of baker's yeast manufacture
- proof time lower is at least 10 % shorter than the proof time measured under the same test conditions using the control yeast,
- absence of any bad taste or any off-flavors.

The tests carried out with the dough pieces corresponding to the formulations of sweet rolls make it possible to confirm the selection because they permit to verify that the desired properties of the new yeasts manifest themselves in a wide range of sweet pastries.

Two particularly advantageous strains have been selected at the end of the series of these three systematic selection tests: the strains deposited at the CNCM under the numbers I-2421 and I-2422.

These two strains have been selected because yeasts manufactured starting from these strains gave rise with respect to the control yeast to the greatest differences in all the tests on frozen and thawed doughs.

The yeasts obtained with these two selected strains permitted to obtain frozen sweet-Danish-pastry dough pieces which, when thawed after 100 days at - 20°C, provided a total amount of gas released measured with the zymotachygraphe CHOPIN^{®} which was at least 30 % higher than the total amount of gas released measured under the same test conditions using the control yeast and a proof time which was at least 20 % shorter, in fact 25 % shorter, than the proof time measured under the same test conditions using the control yeast.

These two strains permit to obtain yeast creams and fresh compressed yeasts which show a good aptitude to retain their properties during storage.

These two strains also have the merit of giving baker's yeasts having very good general performances in frozen French style doughs, i.e. in the manufacture of French type breads (breads without addition of sugar and fat to the flour) in the framework of delayed bread-making processes. The said very good general performances are performances which are at least equal to those of the best known baker's yeasts for this type of use.

These two selected strains CNCM I-2421 and I-2422 also present the interesting properties consisting in the fact that they respond well to cultivation processes with discontinuous inflow of molasses such as those disclosed in the UK patent No. 1,539,211 or US patent No. 4,328,250 with the view of improving the performances of the yeasts on sweet doughs and especially in the fact that they retain these improved performances on sweet dough, due to the use of the said cultivation processes, in sweet dough pieces thawed after several months of storage at - 20°C.

The baker's yeasts according to the invention are preferably obtained using this kind of cultivation processes for adaptation to the fermentation of sweetened doughs.

These two strains CNCM I-2421 and I-2422 also have the property to provide frozen intermediate dry yeasts, the use of which is particularly interesting for the making of frozen doughs.

The frozen intermediate active dry yeasts are defined as frozen dry yeasts in the form of particles, having an intermediate dry matter content, i.e. a dry matter content of from 70 to 80% in weight, preferably 72 to 78%, more preferably 74 to 78%. The said frozen intermediate active dry yeasts can be made as described in European patent No. 0237427 B2 corresponding to Canadian patent No. 1,299,435 or corresponding to Australian patent No. 609 030 (document No. AU-B-69781/87), the entire disclosures of which are incorporated by reference.

Different trials carried out with strain CNCM I-2421 cultivated according to a process with discontinuous inflow of molasses during the whole or part of the last cycle of multiplication, have led to frozen intermediate dry yeasts having between 70 and 80 % dry matter, preferably between 72 and 78 % dry matter, and giving the following total quantities of gas produced in tests A₁, A₅, A₆:
test A₁ 170 ml to 190 ml in two hours
test A₅ 110 ml to 130 ml in two hours
test A₆ 115 ml to 140 ml in two hours.

In these tests A₁, A₅ and A₆ carried out with frozen intermediate active dry yeasts, the amounts of frozen intermediate active dry yeast corresponding to 160 or 106 mg of yeast solids content (tests A₁ or A₅) or to 320 mg of yeast solids content (test A₆) are thawed during one hour at room temperature before being mixed with the 15 ml of water as described column 5 of US patent No. 5,741,695.

These two strains CNCM I-2421 and I-2422 can be characterized using the identification technique of yeast strains using the Polymerase Chain Reaction and based on the amplification of the inter delta zones of the retrotransposon TY1, and which is disclosed in the Article "Identifications of Yeast Strains Using the Polymerase Chain Reaction" by F. Ness, F. Lavallée, D. Dubourdieu, M. Aigle and L. Dulau, published in J. Sci. Food Agric. 1993, 62, 89-94.

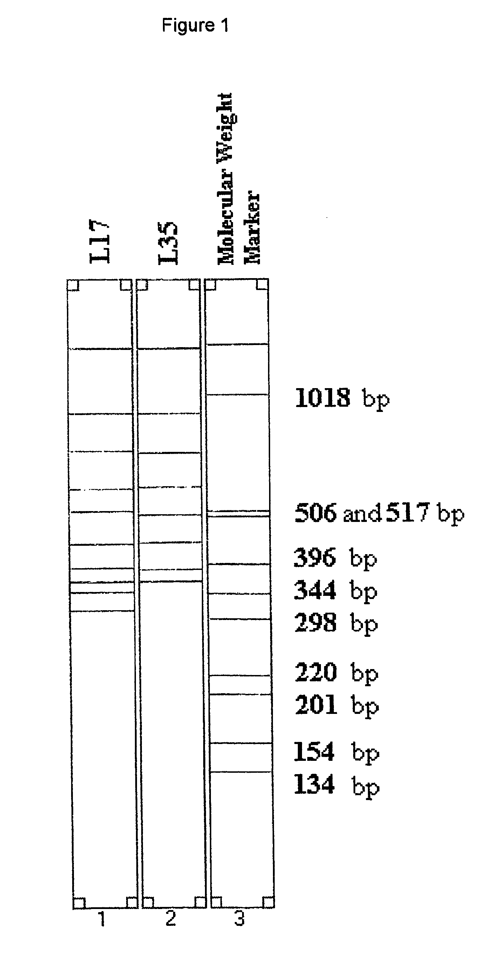

Figure 1 shows in 1, 2 and 3 the electrophoresis profiles of amplified DNA sequences of strains L17 and L35 and a profile of digested DNA used as a molecular weight marker. It is apparent from Figure 1 that the strain L17 (CNCM I-2421) presents two supplemental bands with respect to the strain L35 (CNCM I-2422).

The invention thus relates to the strains deposited under the numbers CNCM I-2421 and I-2422.

The invention also relates to the use of the two strains CNCM I-2421 and CNCM I-2422, for the preparation of baker's yeasts, to baker's yeasts obtained by the cultivation of said strains, and to the use of the said baker's yeasts in the preparation of doughs and baked products.

The baker's yeast according to the invention can be in the form of yeast cream, in the form of fresh compressed yeast, in the form of active dried yeast, and preferably in the form of frozen intermediate active dry yeast.

As already indicated, baker's yeast strains which have the property to be resistant to the stress caused by freezing were known, but these known baker's yeast strains cannot be used without formation of off-flavors in the manufacture of bakery products containing cinnamon.

This disadvantageous property can be corrected and is corrected by the deletion or disruption or inactivation in the genome of said baker's yeast strains of the PAD1 gene(s) encoding phenylacrylic acid decarboxylase, the enzyme which enables the decomposition of cinnamic acid by the yeast.

The PAD1 gene is entirely described and characterized by Clausen et al. (1994), Gene 142: 107-112 in an article entitled "PAD1 encodes phenylacrylic acid decarboxylase which confers resistance to cinnamic acid in Saccharomyces cerevisiae." This PAD1 gene is also called Phenolic Off Flavors gene or POF gene.

Such a targeted inactivation of the PAD1 gene can be obtained by conventional gene replacement methods (see Rothstein in Guthrie C. and Fiur GR (editors) Guide to Yeast Genetics and Molecular Biology in Methods in Enzymology vol. 194: 281-301) or by employing an integration/excision DNA cassette as described in the European patent application No. 0 994 192 or in the corresponding US patent application Serial No. 09/415,216 filed on October 12th, 1999 and having as title "Yeast transformation cassette" or still the Australian patent application No. 3572/99. The above patent applications disclose integration/excision DNA cassettes which permit the total or partial deletion of a same gene in Saccharomyces cerevisiae, leaving in the host strain only yeast DNA. In order to disrupt the PAD1 gene(s), an integration/disruption cassette(s) CAS-PAD is constructed on the basis of the same principle as the integration/disruption cassettes CAS-SUC disclosed in the example 4 of these patent applications and the said cassette(s) CAS-PAD is(are) used to delete or disrupt the alleles of the PAD1 gene by integration(s)/excision(s) in a manner analogous as disclosed in the said example 4. This strategy consisting in the use of the said integration/excision DNA cassette(s) is preferred as it allows to inactivate all copies of the PAD1 gene in a strain containing more than one copy thereof, as is often the case in industrial strains. This strategy furthermore leads to the construction of strains, in which the selectable marker has been eliminated.

The elimination of the selectable marker is very important. To date no baker's yeast is obtained on an industrial level by multiplication of a genetically modified strain and it is generally admitted that only a stable genetically modified strain without marker could be acceptable as a commercial baker's yeast strain.

The invention relates to the new baker's yeast strains obtained (or modified) by clean deletion of the PAD1 gene(s) encoding phenylacrylic acid decarboxylase, to the use of said strains for the preparation of baker's yeasts, to baker's yeasts obtained by the cultivation of said strains, and to the use of said baker's yeasts in the preparation of doughs and baked products.

A clean gene deletion or inactivation is defined as a genetic modification which cuts out the expression of the deleted or inactivated gene(s) (i.e. in the frame of present invention the PAD1 gene(s)), without leading to the expression of a heterologous gene, and preferably without leading to the production of any new compound as compared to a natural yeast mutant strain which has its said PAD1 gene(s) disrupted or inactivated.

Another preferred strategy for obtaining baker's yeast strains which have their PAD1 gene(s) inactivated, consists in mutating baker's yeast strains intended to be modified, or preferably their segregants, by a classic mutagenic treatment giving a cell survival ratio of about 20 to 30%. In a first step, those mutated strains are selected which show no phenylacrylic acid decarboxylase activity in the presence of cinnamic acid and/or ferulic acid and/or coumaric acid, and in particular which show no production of off-flavors (no production of volatile phenols and/or styrene) in the presence of these ingredients. It is then verified that the said mutated (mutant) strains, possibly obtained after conjugation of mutated segregants, which do not express a functional pad1 protein from the PAD1 gene(s) have retained the properties of the initial strain which are of interest with respect to the use of the strains as baker's yeast strains.

A preferred embodiment of the invention relates to baker's yeast strains resistant to the stress caused by freezing and modified by clean deletion (disruption) or inactivation of the PAD1 gene(s) which deletion or inactivation cuts out the expression of the deleted or inactivated gene(s) without leading to the expression of a heterologous gene.

The starting or host strains are preferably baker's yeast strains, such as known baker's yeast strains, which are already resistant to the stress caused by freezing, such as the baker's yeast strains giving the European baker's yeasts having good performance on frozen doughs cited hereabove. The said host baker's yeast strains are modified by clean deletion of the PAD1 gene(s).

Preferably, the said starting or host baker's yeast strains are such that they pass successfully the first selection test and the third selection test hereabove disclosed, and the modified strains pass successfully the three selection tests hereabove disclosed.

The invention relates also to the same or equivalent baker's yeast strains obtained by mutation of the gene(s) PAD1 which is(are) inactivated. Indeed, the said mutation process leads to a clean inactivation as hereabove defined. Indeed, the invention relates to all the new baker's yeast strains obtained by clean inactivation of their PAD1 gene(s) whatever the used means of inactivation.

The invention relates also to new baker's yeasts having the new properties hereabove disclosed, obtained by a production process using any of the said new modified or mutated baker's yeast strains hereabove disclosed.

The invention also relates to the use of the hereabove disclosed modified or mutated baker's yeast strains, obtained by inactivation of the PAD1 gene(s), for the preparation of baker's yeasts in the form of yeast creams, fresh compressed yeasts, frozen intermediate active dry yeasts, and active dry yeasts.

The invention also relates to a process for the preparation of new baker's yeasts according to the invention comprising the conventional cultivation or multiplication of a selected starting strain according to the invention.

Preferably, the new baker's yeasts according to the invention are obtained by the use of a special cultivation process corresponding to a fed-batch process comprising several consecutive cycles of cultivation with a discontinuous inflow of molasses during the whole or part of the last cycle of cultivation.

Another object of the invention is new baker's yeasts having the new properties hereabove disclosed and obtained by or obtainable by a process comprising a discontinuous inflow of molasses during the whole or part of the last cycle of cultivation.

Preferably the said selected starting strain is strain CNCM I-2421 or I-2422, but the said selected strain can also be a strain similar to strains CNCM I-2421 and CNCM I-2422, i.e. a strain sharing all the properties common to these two strains and/or able to be selected in the manner hereabove disclosed, and/or a baker's yeast strain obtained by clean inactivation of its PAD1 gene(s), preferably resistant to the stress caused by freezing.

The invention also relates to new frozen intermediate active dry yeast products, having between 70 and 80% dry matter, preferably between 72 and 78% dry matter, more preferably between 74 and 78% dry matter. The said frozen intermediate active dry yeasts are preferably in the form of rod shaped flowing particles of a diameter of less than 3mm, preferably less than 1mm. They are preferably obtained by gentle drying of fresh baker's yeast (i.e. a baker's yeast having between about 30% and about 35% dry matter) until the desired dry matter and a freezing by fluidization. The said frozen intermediate active dry yeasts preferably have the following properties:
- very good performances when used as described above in tests A₁, A₅ and A₆ as defined above, carried out with the fermentometer of Burrows and Harrison, i.e.
   in test A₁: total quantity of gas produced between 170 ml and 190 ml in two hours,
   in test A₅: total quantity of gas produced between 110 ml and 130 ml in two hours,
   in test A₆: total quantity of gas produced between 115 ml and 140 ml in two hours;
- they are resistant to the stress caused by freezing when they are used in sweet frozen doughs, i.e. when they are used for making sweet-Danish-pastry doughs comprising 18% sucrose with respect to the flour used and comprising fats, the following characteristics are obtained:
   - the total amount of gas released recorded with the zymotachygraphe Chopin^{®} on a dough piece frozen and thawed after at least 100 days is at least 20% higher than the total amount of gas released recorded under the same conditions when using the same amount of yeast dry matter of a conventional control fresh yeast obtained starting from strain CNCM I-2412, and the proof time of the said sweet-Danish-pastry doughs, frozen and thawed after at least 100 days is at least 10% shorter than the proof time measured under the same conditions when using the same amount of yeast dry matter of the said conventional control fresh yeast;
- they do not give rise to the appearance of bad taste or of off-flavors in the presence of cinnamon.

The said new frozen intermediate active dry yeasts in the form of fine free flowing particles can contain free flowing agents or anticaking agents such as silica and silicates. It can also contain drying processing aids and/or rehydration agents such as sorbitan monostearate, gums, carboxy-methyl-cellulose.

The said new frozen intermediate active dry yeasts according to the invention have the advantage substantially to retain their initial properties during at least 6 months, preferably during at least one year.

The invention thus relates to new baker's yeasts according to the invention in the form of particles of frozen active dry yeast having an intermediate dry matter content, i.e. a dry matter content between 70% and 80%, preferably between 72% and 78%. Preferably the said new baker's yeasts in the form of frozen active dry yeast are obtained by or obtainable by a process comprising the use as starting strain of one of the strains belonging to the group of the strains CNCM I-2421 and I-2422 and the strains similar to these two strains CNCM I-2421 and I-2422 and the baker's yeast strains obtained by inactivation of the PAD1 gene(s). Advantageously, the said process comprises a discontinuous inflow of molasses during the whole or part of the last cycle of multiplication. Preferably the new baker's yeasts according to the invention, in the form of frozen intermediate dry yeasts, have the hereabove defined gas releases in the hereabove defined tests A₁, A₅ and A₆.

The invention also relates to a process for the preparation of baker's yeasts comprising the use as starting strain of one of the strains of the group comprising the strains deposited according to the Budapest Convention with the "Collection Nationale de Cultures de Microorganismes", Institut Pasteur, 28 rue du Docteur Roux, 75724 PARIS CEDEX 15, under the numbers I-2421 and I-2422.

Preferably in the said process, the strain chosen among the group comprising the strains I-2421 and I-2422 and the hereabove defined strains obtained by clean inactivation of the PAD1 gene(s) is cultivated according to a fed-batch process comprising a discontinuous inflow of molasses during the whole or part of the last cycle of cultivation, i.e. during at least part of the last cultivation stage before the harvesting of the yeast cells in order to obtain commercial cream yeast, fresh compressed or crumbled yeast, active dry yeast or intermediate frozen active dry yeast.

The process according to the invention for the preparation of new baker's yeasts according to the invention is original due to the use of a selected starting strain as hereabove disclosed. Preferably the said process uses a cultivation process comprising a discontinuous inflow of molasses.

Concerning any details relating to these techniques, reference is made to the manual "YEAST TECHNOLOGY", Reed and Peppler, The AVI PUBLISHING, 1973, or to the second edition of that manual by Reed and Nagodawithana, an AVI book published by VAN NOSTRAND REINHOLD, 1991, which are incorporated by reference, and to US patents 4,328,250, 4,396,632 and 5,741,695, UK patent 1,539,211, European patent No. 0 237 427 B2 and Australian patent No. 609030, the entire disclosures of which are also incorporated by reference.

Indeed, the invention also relates to new baker's yeasts obtained or able to be obtained (= obtainable) according to the hereabove disclosed processes.

The invention equally relates to the use of baker's yeasts according to the invention for the manufacture of doughs aromatized or flavored with cinnamon and/or for the manufacture of frozen dough pieces, in particular of sweet doughs. The invention thus relates, on the one hand, to processes for the manufacture of doughs aromatized with cinnamon, and, on the other hand, to processes for the manufacture of frozen dough pieces, in particular sweet frozen dough pieces, comprising the use of baker's yeasts according to the invention. Preferably the invention relates to the said dough making processes comprising the use of a baker's yeast belonging to the group of the new baker's yeasts which show good general performances in not delayed bread-makings, which are resistant to the stress caused by freezing when they are used in sweetened doughs, and which do not give rise to the appearance of off-flavors in the presence of cinnamon and of the new baker's yeasts obtained or obtainable by the process comprising the use as starting strain of one of the strains of the group comprising the strains CNCM I-2421 and I-2422, and/or which are able to be selected as disclosed hereabove, and the baker's yeast strains obtained by clean deletion or inactivation of the PAD1 gene(s) which deletion or inactivation cuts out the expression of the deleted or inactivated gene(s), without leading to the expression of a heterologous gene , it being generally understood that these modified or mutated baker's yeast strains are preferably also resistant to the stress caused by freezing, and more preferably pass the three selection tests hereabove disclosed. Preferably, these new baker's yeasts are in the form of frozen intermediate active dry yeasts.

## Claims

1. Baker's yeast strain deposited according to the Budapest Convention with the "Collection Nationale de Cultures de Microorganismes", Institut Pasteur, 28 rue du Docteur Roux, 75724 PARIS CEDEX 15, FRANCE, under the number I-2421.

2. Baker's yeast strain deposited according to the Budapest Convention with the "Collection Nationale de Cultures de Microorganismes", Institut Pasteur, 28 rue du Docteur Roux, 75724 PARIS CEDEX 15, FRANCE, under the number I-2422.

3. Baker's yeast strain resistant to the stress caused by freezing and modified by clean deletion or inactivation of the PAD1 gene(s) which deletion or inactivation cuts out the expression of the deleted or inactivated gene(s) without leading to the expression of a heterologous gene.

4. Baker's yeasts obtained by cultivation of the strain deposited according to the Budapest Convention with the "Collection Nationale de Cultures de Microorganismes", Institut Pasteur, 28 rue du Docteur Roux, 75724 PARIS CEDEX 15, under the number I-2421.

5. Baker's yeasts obtained by cultivation of the strain deposited according to the Budapest Convention with the "Collection Nationale de Cultures de Microorganismes", Institut Pasteur, 28 rue du Docteur Roux, 75724 PARIS CEDEX 15, under the number I-2422.

6. Baker's yeasts obtained by the cultivation of a strain resistant to the stress caused by freezing and modified by clean deletion or inactivation of the PAD1 gene(s) which deletion or inactivation cuts out the expression of the deleted or inactivated gene(s) without leading to the expression of a heterologous gene.

7. Baker's yeasts according to any one of claim **4** to **6,** obtained by a process comprising the cultivation of a starting strain according to a fed-batch process comprising several consecutive cycles of cultivation with a discontinuous inflow of molasses during the whole or part of the last cycle of cultivation.

8. Baker's yeasts obtained by a process:
- comprising the use as starting strain of a strain selected from the group consisting of the strains deposited according to the Budapest Convention with the "Collection Nationale de Cultures de Microorganismes", Institut Pasteur, 28 rue du Docteur Roux, 75724 PARIS CEDEX 15, under the numbers I-2421 and 1-2422 and baker's yeast strains resistant to the stress caused by freezing and obtained by clean deletion or inactivation of the PAD 1 gene(s) which deletion or inactivation cuts out the expression of the deleted or inactivated gene(s) without leading to the expression of a heterologous gene, and
- comprising several consecutive cycles of cultivation with a discontinuous inflow of molasses during the whole or part of the last cycle of multiplication of the selected strain.

9. Baker's yeasts according to any one of claim **4** to **7,** in the form of a frozen intermediate active dry yeast product having between 70 and 80 % dry matter, preferably between 72 and 78 % dry matter, and more preferably between 74 and 78%.

10. Process for the preparation of baker's yeasts, comprising the use as starting strain of the strain according to one of the claims **1** to **3.**

11. Process for the preparation of baker's yeasts according to claim **10,** wherein the said starting strain is cultivated according to a fed batch process comprising a discontinuous inflow of molasses during the whole or part of the last cycle of cultivation.

12. Process for the manufacture of doughs aromatized with cinnamon comprising the use of a baker's yeast according to any one of claims **4** to **9.**

13. Process for the manufacture of frozen sweet doughs pieces comprising the use of a new baker's yeast according to any one of claims **4** to **9.**

14. Process for the production of bread-making doughs according to one of claims **12** and **13** wherein the baker's yeast is used in the form of a frozen intermediate dry yeast product.

## Patentansprüche

1. Bäckerhefestamm hinterlegt gemäß des Budapester Vertrags bei der "Collection Nationale de Cultures de Microorganismes", Institut Pasteur, 28 rue du Docteur Roux, 75724 PARIS CEDEX 15, FRANKREICH, unter der Nummer I-2421.

2. Bäckerhefestamm hinterlegt gemäß des Budapester Vertrags bei der "Collection Nationale de Cultures de Microorganismes", Institut Pasteur, 28 rue du Docteur Roux, 75724 PARIS CEDEX 15, FRANKREICH, unter der Nummer I-2422.

3. Bäckerhefestamm, der resistent ist gegen den Stress verursacht durch Gefrieren und der modifiziert ist durch saubere Deletion oder Inaktivierung des/der PAD1-Gens/Gene, wobei die Deletion oder Inaktivierung die Expression des/der deletierten oder inaktivierten Gens/Gene ausschaltet, ohne zur Expression eines heterologen Gens zu führen.

4. Bäckerhefen erhältlich durch Kultivierung des Stamms hinterlegt gemäß des Budapester Vertrags bei der "Collection Nationale de Cultures de Microorganismes", Institut Pasteur, 28 rue du Docteur Roux, 75724 PARIS CEDEX 15, unter der Nummer I-2421.

5. Bäckerhefen erhältlich durch Kultivierung des Stamms hinterlegt gemäß des Budapester Vertrags bei der "Collection Nationale de Cultures de Microorganismes", Institut Pasteur, 28 rue du Docteur Roux, 75724 PARIS CEDEX 15, unter der Nummer I-2422.

6. Bäckerhefen erhältlich durch die Kultivierung eines Stamms, der resistent ist gegen den Stress verursacht durch Gefrieren und der modifiziert ist durch saubere Deletion oder Inaktivierung des/der PAD1-Gens/Gene, wobei die Deletion oder Inaktivierung die Expression des/der deletierten oder inaktivierten Gens/Gene ausschaltet, ohne zur Expression eines heterologen Gens zu führen.

7. Bäckerhefen gemäß einem der Ansprüche **4** bis **6,** erhältlich durch ein Verfahren umfassend die Kultivierung eines Ausgangsstamms gemäß einem Fed-Batch-Verfahren umfassend mehrere aufeinander folgende Kultivierungszyklen mit einem diskontinuierlichen Zulauf von Melasse während des ganzen oder eines Teils des letzten Kultivierungszyklus.

8. Bäckerhefen erhältlich durch ein Verfahren:
- umfassend die Verwendung eines Stamms als Ausgangsstamm, ausgewählt aus der Gruppe bestehend aus den Stämmen, die gemäß des Budapester Vertrags bei der "Collection Nationale de Cultures de Microorganismes", Institut Pasteur, 28 rue du Docteur Roux, 75724 PARIS CEDEX 15, unter den Nummern I-2421 und I-2422 hinterlegt sind und Bäckerhefestämmen, die resistent sind gegen Stress verursacht durch Gefrieren und erhältlich sind durch saubere Deletion oder Inaktivierung des/der PAD1-Gens/Gene, wobei die Deletion oder Inaktivierung die Expression des/der deletierten oder inaktivierten Gens/Gene ausschaltet, ohne zur Expression eines heterologen Gens zu führen, und
- umfassend mehrere aufeinander folgende Kultivierungszyklen mit einem diskontinuierlichen Zulauf von Melasse während des ganzen oder eines Teils des letzten Vermehrungszyklus des ausgewählten Stamms.

9. Bäckerhefen gemäß einem der Ansprüche **4** bis **7,** in der Form eines gefrorenen intermediären aktiven Trockenhefeproduktes, das zwischen 70 und 80% Trockenmasse hat, bevorzugt zwischen 72 und 78% Trockenmasse, und stärker bevorzugt zwischen 74 und 78%.

10. Verfahren zur Herstellung von Bäckerhefen, umfassend die Verwendung des Stamms gemäß einem der Ansprüche **1** bis **3** als Ausgangsstamm.

11. Verfahren zur Herstellung von Bäckerhefen gemäß Anspruch **10,** worin der Ausgangsstamm gemäß einem Fed-Batch-Verfahren umfassend einen diskontinuierlichen Zulauf von Melasse während des ganzen oder eines Teils des letzten Kultivierungszyklus kultiviert wird.

12. Verfahren zur Herstellung von Teigen, die mit Zimt aromatisiert sind, umfassend die Verwendung einer Bäckerhefe gemäß einem der Ansprüche **4** bis **9.**

13. Verfahren zur Herstellung von gefrorenen süßen Teigstücken umfassend die Verwendung einer neuen Bäckerhefe gemäß einem der Ansprüche **4** bis **9.**

14. Verfahren zur Herstellung von Teigen zur Brotbereitung gemäß einem der Ansprüche **12** und **13,** worin die Bäckerhefe in der Form eines gefrorenen intermediären Trockenhefeproduktes verwendet wird.

## Revendications

1. Souche de levure de boulanger déposée selon la Convention de Budapest à la "Collection Nationale de Cultures de Micro-organismes", Institut Pasteur, 28 rue du Docteur Roux, 75724 PARIS CEDEX 15, FRANCE, sous le numéro I-2421.

2. Souche de levure de boulanger déposée selon la Convention de Budapest à la "Collection Nationale de Cultures de Micro-organismes", Institut Pasteur, 28 rue du Docteur Roux, 75724 PARIS CEDEX 15, FRANCE, sous le numéro I-2422.

3. Souche de levure de boulanger résistante au stress entraîné par la congélation et modifiée par délétion propre ou inactivation du (des) gène(s) PAD1, cette délétion ou inactivation supprimant l'expression du ou des gènes supprimés ou inactivés sans entraîner l'expression d'un gène hétérologue.

4. Levures de boulanger obtenues par culture de la souche déposée selon la Convention de Budapest à la "Collection Nationale de Cultures de Micro-organismes", Institut Pasteur, 28 rue du Docteur Roux, 75724 PARIS CEDEX 15, FRANCE, sous le numéro I-2421.

5. Levures de boulanger obtenues par culture de la souche déposée selon la Convention de Budapest à la "Collection Nationale de Cultures de Micro-organismes", Institut Pasteur, 28 rue du Docteur Roux, 75724 PARIS CEDEX 15, FRANCE, sous le numéro I-2422.

6. Levures de boulanger obtenues par la culture d'une souche résistante au stress entraîné par la congélation et modifiée par délétion propre ou inactivation du (des) gène(s) PAD1, cette délétion ou inactivation supprimant l'expression du ou des gènes supprimés ou inactivés sans entraîner l'expression d'un gène hétérologue.

7. Levures de boulanger selon l'une quelconque des revendications 4 à 6, obtenues par un procédé comprenant la culture d'une souche de départ selon un procédé en fed-batch comprenant plusieurs cycles de culture consécutifs avec un apport discontinu de mélasse pendant tout ou partie du dernier cycle de culture.

8. Levures de boulanger obtenues par un procédé:
- comprenant l'utilisation comme souche de départ d'une souche choisie dans le groupe constitué par les souches déposées selon la Convention de Budapest à la "Collection Nationale de Cultures de Micro-organismes", Institut Pasteur, 28 rue du Docteur Roux, 75724 PARIS CEDEX 15, FRANCE, sous les numéros I-2421 et 1-2422 et des souches de levures de boulanger résistantes au stress entraîné par la congélation et modifiées par délétion propre ou inactivation du (des) gène(s) PAD1, cette délétion ou inactivation supprimant l'expression du ou des gènes supprimés ou inactivés sans entraîner l'expression d'un gène hétérologue, et
- comprenant plusieurs cycles de culture consécutifs avec un apport discontinu de mélasse pendant tout ou partie du dernier cycle de multiplication de la souche choisie.

9. Levures de boulanger selon l'une quelconque des revendications 4 à 7, sous forme d'un produit de type levure sèche active intermédiaire surgelée ayant entre 70 et 80 % de matière sèche, de préférence entre 72 et 78 % de manière sèche, et de façon plus préférable entre 74 et 78 %.

10. Procédé de préparation de levures de boulanger, comprenant l'utilisation comme souche de départ de la souche selon l'une des revendications 1 à 3.

11. Procédé de préparation de levures de boulanger selon la revendication 10, dans lequel ladite souche de départ est cultivée selon un procédé en fed-batch comprenant un apport discontinu de mélasse pendant tout ou partie du dernier cycle de culture.

12. Procédé de fabrication de pâtes aromatisées à la cannelle, comprenant l'utilisation d'une levure de boulanger selon l'une quelconque des revendications 4 à 9.

13. Procédé de fabrication de morceaux de pâte sucrée surgelés, comprenant l'utilisation d'une nouvelle levure de boulanger selon l'une quelconque des revendications 4 à 9.

14. Procédé de production de pâtes pour la fabrication de pain selon l'une des revendications 12 et 13, dans lequel on utilise la levure de boulanger sous forme d'un produit de type levure sèche intermédiaire surgelée.
